# EUROPEAN PATENT SPECIFICATION

(11) **EP 2 928 460 B1**
(45) Date of publication and mention of the grant of the patent: **27.12.2017**
(21) Application number: 13860898.9
(22) Date of filing: 21.06.2013
(51) Int. Cl.: A61K 9/06, A61K 9/08, A61K 31/194, A61K 47/10, A61K 47/46, A61P 17/00, A61P 17/02, A61P 17/04, A61P 17/06, A61P 17/08, A61P 17/10, A61P 17/14

(54) **METHODS FOR PREVENTING AND TREATING INFLAMMATORY SKIN CONDITIONS**
VERFAHREN ZUR VORBEUGUNG UND BEHANDLUNG ENTZÜNDLICHER HAUTERKRANKUNGEN
PROCÉDÉS POUR LA PRÉVENTION ET LE TRAITEMENT D'AFFECTIONS CUTANÉES INFLAMMATOIRES

(30) Priority: 06.12.2012 US 201213694484
(43) Date of publication of application: 14.10.2015
(73) Proprietor: Aurin Biotech Inc., Vancouver, British Columbia V6T 1C1 (CA)
(72) Inventor: MCGEER, Patrick, L., Vancouver, British Columbia V6T 1C1 (CA); LEE, Moonhee, Vancouver, British Columbia V6T 1R9 (CA); BELL, Douglas, N., Rancho Mirage, California 92270 (US)
(74) Representative: Asensio, Raffaella Consuelo
(86) International application number: PCT/CA2013/050488
(87) International publication number: WO 2014/085920

(56) References cited:
- WO-A1-2010/042728
- WO-A2-01/57184
- CA-A1- 2 388 924
- US-A- 4 007 270
- US-A1- 2013 035 388
- US-A1- 2013 035 392
- US-B1- 6 248 365
- LEE ET AL.: 'Selective inhibition of the membrane attack complex of complement by low molecular weight components of the aurin tricarboxylic acid synthetic complex' NEUROBIOLOGY OF AGING vol. 33, no. 10, October 2012, pages 2237 - 2246, XP055239962
- LAPIDUS ET AL.: 'New inhibitors of complement fixation' IMMUNOPHARRNACOLOGY vol. 3, 1981, pages 137 - 145, XP023816027
- KOTNIK: 'Complement in skin diseases' ACTA DEMATOVEN. vol. 20, no. 1, 2011, pages 3 - 11, XP055257139
- TAGAMI: 'The role of complement-derived mediators in inflammatory skin diseases' ARCHIVES IN DERMATOLOGICAL RESEARCH vol. 284, no. 1, 1992, pages S2 - S9, XP008179402

## Description

### Technical Field

This invention pertains to means to treat inflammatory skin conditions.

### Background

Inflammatory skin conditions can have significant physical and psychological impacts on the quality of life of those afflicted. Inflammatory skin conditions include inflammatory skin disorders such as acne vulgaris, allergic or atopic dermatitis, alopecia areata, androgenetic alopecia, dandruff, dermatitis herpetiformis, discoid lupus erythematosus, pemphigus, primary cicatricial alopecia, psoriasis, and seborrheic dermatitis. Inflammatory skin conditions can also include skin inflammation due to exposure to excessive thermal and ultraviolet radiation. Methods for preventing and/or treating such inflammatory skin conditions are desirable.

Document US 6248365 discloses the use of complement inhibitors (eg C1 inactivator, factor I and factor H) for the treatment of skin inflammatory disorders.

### Summary

The invention provides aurin tricarboxylic acid (ATA), aurin quadracarboxylic acid (AQA), aurin hexacarboxylic acid (AHA), or a combination thereof for use as an active agent for topical treatment of an inflammatory skin condition, wherein said aurin tricarboxylic acid (ATA), aurin quadracarboxylic acid (AQA), aurin hexacarboxylic acid (AHA), and/or a combination thereof have a molecular weight less than 1kDa. The combination may comprise 60 to 90% AHA, 10 to 30% AQA, and 1 to 20% AHA, or comprise 78% AHA, 15% AQA, and 7% AHA. The condition may be acne vulgaris, allergic or atopic dermatitis, alopecia areata, androgenetic alopecia, dandruff, dermatitis herpetiformis, discoid lupus erythematosus, pemphigus, primary cicatricial alopecia, psoriasis, seborrheic dermatitis, thermal or ultraviolet burns, or any condition affecting the skin involving abnormal activation of complement. The therapeutic agent may be formulated in a hair care preparation, skin care preparation, or a pharmaceutical composition at a concentration of 0.1 to 100 mg/mL. The hair care preparation, skin care preparation or pharmaceutical composition may be a spray, gel, cream, lotion, stick, ointment, scrub, soap bar, tonic, roll-on formulation, sunscreen, shampoo or mousse.

### Brief Description of the Drawings

In drawings which show non-limiting embodiments of the invention:
Figures 1A-1D are scanned images illustrating the effect of an embodiment of the invention on androgenetic alopecia. Figures 1A and 1C show affected scalps of male patients pre-treatment, and Figures 1B and ID show the respective scalps of the male patients post-treatment.
Figures 2A and 2B are scanned images illustrating the effect of an embodiment of the invention on acne vulgaris. Figure 2A shows an affected facial area of a male patient pre-treatment, and Figure 2B shows the same area of the male patient post-treatment.
Figures 3A and 3B are scanned images illustrating the effect of an embodiment of the invention on sunburns. Figure 3A shows a sunlight exposed area of a female patient pre-treatment, and Figure 3B shows the same area of the female patient post-treatment.
Figures 4A and 4B are scanned images illustrating the effect of an embodiment of the invention on allergic or atopic dermatitis. Figure 4A shows an affected area of the abdomen of a male patient pre-treatment, and Figure 4B shows the same area of the male patient post-treatment.

### Detailed Description

Throughout the following description, specific details are set forth in order to provide a more thorough understanding of the invention.

The invention relates to aurin tricarboxylic acid (ATA), quadracarboxylic acid (AQA), aurin hexacarboxylic acid (AHA) and/or combinations thereof (ATAC) having a molecular weight less than 1kDa (collectively, "low molecular weight ATA compounds") for use as an active agent for topical treatment of an inflammatory skin condition. In some embodiments the conditions treated involve self-damaging complement activation. The term "inflammatory skin condition" as used herein refers to a skin or scalp condition characterized by one or more of irritation, blistering, redness, flaking, localized heat, pain, itching, and hair follicle damage/destruction.

ATA has an approximate molecular weight of 422. AQA has an approximate molecular weight of 573. AHA has an approximate molecular weight of 857. ATA, AQA, and AHA are low molecular weight components of crude synthetic "ATA" or commercial "ATA" (e.g. aluminon), and can for example be obtained from these sources by passing them through a 1 kDa filter. ATAC comprises a mixture of ATA, AQA and AHA and may in example embodiments be comprised of approximately 78% ATA, 15% AQA, and 7% AHA. In some embodiments, ATAC may be comprised of 60 to 90% ATA, 10 to 30% AQA, and 1 to 20% AHA.

According to some embodiments, low molecular weight ATA compounds are topically administered to patients with inflammatory skin conditions such as acne vulgaris, allergic dermatitis, alopecia areata, androgenetic alopecia, atopic dermatitis, dandruff, dermatitis herpetiformis, discoid lupus erythematosus, pemphigus, primary cicatricial alopecia, psoriasis, seborrheic dermatitis, or ultraviolet or thermal burns.

According to some embodiments, one or more of the low molecular weight ATA compounds may administered in the form of a skin care preparation, hair care preparation or a pharmaceutical composition formulated for application to affected areas of the skin or scalp of a patient. Such preparations or compositions may for example be formulated as a spray, gel, cream, lotion, stick, ointment, scrub, soap bar, tonic, roll-on formulation, sunscreen, shampoo or mousse wherein the one or more low molecular weight ATA compounds, or a pharmaceutically acceptable salt or solvate thereof, is provided in a therapeutically effective amount together with at least one carrier so as to be capable of exerting a therapeutic effect on an inflammatory skin conditions on the patient's skin or scalp. The one or more of the low molecular weight ATA compounds may be provided in such preparations or compositions at a concentration of 0.1 to 100 mg/mL, or 0.1 to 10 mg/mL, or 0.5 to 5 mg/mL, and may be topically applied one to five times daily for example.

In some embodiments, the preparations or compositions may also include one or more of surfactants, propellants, co-solvents, gelling agent, and other ingredients suitable for use in skin or hair care preparations of the type known in the art, such as petrolatum, waxes, oils plasticizers, preservatives, fragrances and the like.

In some embodiments, the preparations or compositions may be applied to the skin or scalp of a patient using bottles or containers (e.g. shampoos), tubes (e.g. gels, creams, ointments, lotions), pressurized canisters (e.g. sprays, mousses), pads, sticks, or "roll-on" applicators (e.g. gels, ointments).

In some embodiments, the preparations or compositions may be formulated to form a film over the skin or scalp, thus allowing controlled release and penetration of the one or more of the low molecular weight ATA compounds into the affected areas.

In some embodiments, one or more of the low molecular weight ATA compounds may be provided at a concentration of 0.1 to 100 mg/mL, or 0.1 to 10 mg/mL, or 0.5 to 5 mg/mL, in an aqueous shampoo base comprising the surfactants sodium lauryl sulfate and cocamidopropyl betaine, for application to the scalp.

In some embodiments, one or more of the low molecular weight ATA compounds may be provided at a concentration of 0.1 to 100 mg/mL, or 0.1 to 10 mg/mL, or 0.5 to 5 mg/mL, in an ointment comprising glycerin, for application to the skin or scalp.

In some embodiments, one or more of the low molecular weight ATA compounds may be provided at a concentration of 0.1 to 100 mg/mL, or 0.1 to 10 mg/mL, or 0.5 to 5 mg/mL, in an gel comprising aloe vera, for application to the skin or scalp.

In some embodiments, one or more of the low molecular weight ATA compounds may be dissolved at a concentration of 0.1 to 100 mg/mL, or 0.1 to 10 mg/mL, or 0.5 to 5 mg/mL, in water and applied as a spray or other means to the affected skin or scalp area.

In some embodiments, one or more of the low molecular weight ATA compounds may be provided at a concentration of 0.1 to 100 mg/mL, or 0.1 to 10 mg/mL, or 0.5 to 5 mg/mL, in a sunscreen formulation comprising one or more known active sunscreen ingredients.

### Example 1 - Androgenetic alopecia

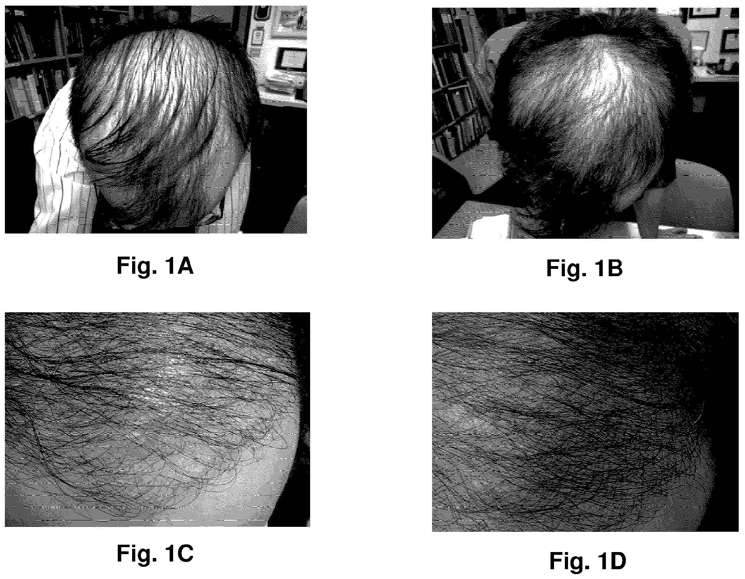

Figure 1A shows the scalp of a male patient suffering from androgenetic alopecia. Figure 1B shows new hair beginning to grow in following 23 days of topical application of an aqueous solution of 1 mg/mL ATAC (approximately 78% ATA, 15% AQA, and 7% AHA) three times per day. Figure 1C shows the scalp of another male patient suffering from androgenetic alopecia. Figure 1D shows new hair growth 2 months following similar application of ATAC.

A further trial conducted applied 1 to 5 mg/mL of ATA, three times daily, to bald areas of a male patient suffering from androgenetic alopecia. An abundance of new hair follicles appeared in the previously bald areas after 4 to 6 weeks of treatment.

### Example 2 - Acne vulgaris

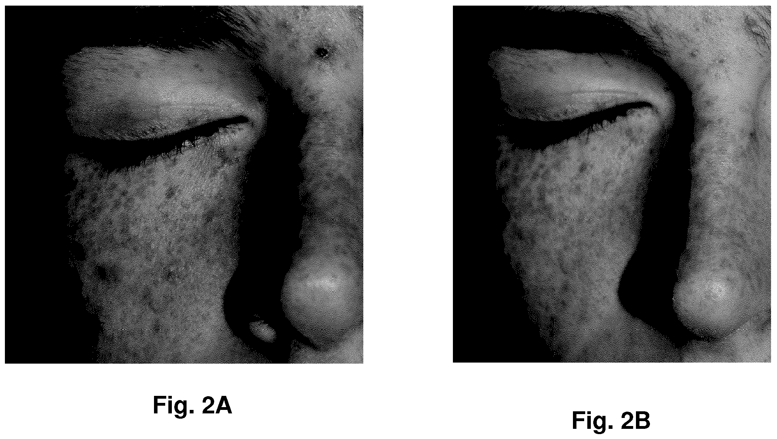

Figure 2A shows a facial area of a young man suffering from acne vulgaris. Figure 2B shows the same area following 7 days of topical application of an aqueous solution of 1 mg/mL ATAC (approximately 78% ATA, 15% AQA, and 7% AHA) three times per day. There is a very noticeable decrease in comedos and erythema in the areas surrounding the comedos. A common, known treatment for acne vulgaris is the antioxidant benzoyl peroxide, which has the drawback of increasing sensitivity to the sun. The present invention is not only effective against acne vulgaris but against sunburn as well, providing a distinct advantage over the commonly utilized benzyol peroxide.

### Example 3 - Thermal and ultraviolet burn

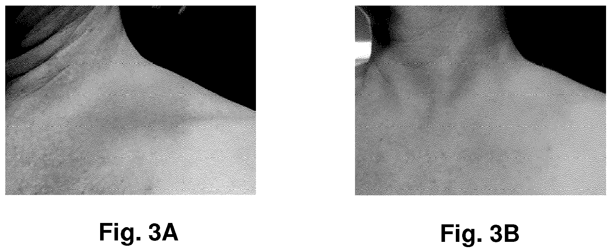

Figure 3A shows an area of sunburn in a woman with sensitive skin that had been briefly exposed to sunlight. Figure 3B shows the same area 25 hours following topical application of an ointment of 1 mg/mL ATAC (approximately 78% ATA, 15% AQA, and 7% AHA) mixed with glycerin. The pain upon touching disappeared almost immediately after application of the ATAC ointment. Redness also *diminished over the next several hours.*

### Example 4 - Allergic or atopic dermatitis

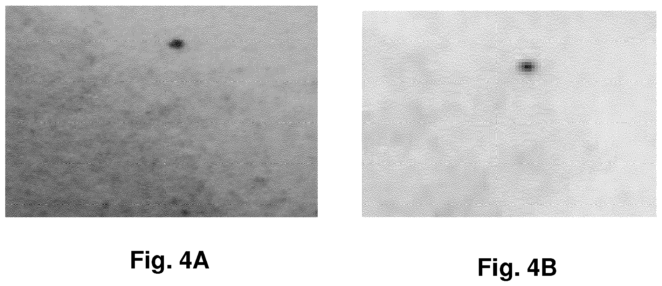

Figure 4A shows an area of the abdomen in a man diagnosed with allergic or atopic dermatitis. Figure 4B shows the same area following topical application of 1 mg/mL ATAC (approximately 78% ATA, 15% AQA, and 7% AHA). Redness decreased and itching disappeared within a few minutes of the application.

### Prophetic Example 1 - Alopecia areata

Alopecia areata is a condition in which there is hair loss, usually from the scalp. It is characterized by an inflammatory response involving lymphocytic infiltration around vulnerable follicles, so that the hair growth disappears. By inhibiting harmful complement activation, low molecular weight ATA compounds will be effective therapeutics. Topically apply a composition comprising 0.1 to 100 mg/mL ATA, AQA, AHA or ATAC to affected areas of the scalp of a patient suffering from alopecia aerata for a period of 1 day to 6 months , from 1 to 5 times daily.

### Prophetic Example 2 - Dandruff and seborrheic dermatitis

Dandruff and seborrheic dermatitis are conditions involving excessive loss of corneocytes from the outer layer of the epidermis. These conditions are characterized by increased levels of inflammatory markers. By inhibiting harmful complement activation, low molecular weight ATA compounds will be effective therapeutics. Topically apply a composition comprising 0.1 to 100 mg/mL ATA, AQA, AHA or ATAC to affected areas of the skin or scalp of a patient suffering from dandruff or seborrheic dermatitis for a period of 1 day to 6 months, from one to five times daily.

### Prophetic Example 3 - Dermatitis herpetiformis

Dermatitis herpetiformis is characterized by an extremely itchy rash, caused by immune attack against the protein epidermal transglutaminase. By inhibiting harmful complement activation, low molecular weight ATA compounds will be effective therapeutics. Topically apply a composition comprising 0.1 to 100 mg/mL ATA, AQA, AHA or ATAC to affected areas of the skin a patient suffering from dermatitis herpetiformis for a period of 1 day to 6 months, from one to five times daily.

### Prophetic Example 4 - Discoid lupus erythematosis

Discoid lupus erythematosis is an autoimmune disorder which is highly exacerbated by sunlight. Known treatment is with topical steroids, indicative of the effectiveness of immune blockade. By inhibiting harmful complement activation, low molecular weight ATA compounds will be effective therapeutics. Topically apply a composition comprising 0.1 to 100 mg/mL ATA, AQA, AHA or ATAC to affected areas of the skin a patient suffering from discoid lupus erythematosis for a period of 1 day to 6 months, from one to five times daily.

### Prophetic Example 5 - Pemphigus

Pemphigus is a potentially fatal disorder where there is an autoimmune attack against desmoglien, the adhesive protein which forms the attachment of adjacent epidermal cells. By inhibiting harmful complement activation, low molecular weight ATA compounds will be effective therapeutics. Topically apply a composition comprising 0.1 to 100 mg/mL ATA, AQA, AHA or ATAC to affected areas of the skin a patient suffering from pemphigus for a period of 1 day to 6 months, from one to five times daily.

### Prophetic Example 6 - Primary cicatricial alopecia

Primary cicatricial alopecia is a skin disorder in which epithelial hair follicle stem cells are damaged or destroyed by inflammation. By inhibiting harmful complement activation, low molecular weight ATA compounds will be effective therapeutics. Topically apply a composition comprising 0.1 to 100 mg/mL ATA, AQA, AHA or ATAC to affected areas of the scalp of a patient suffering from primary cicatricial alopecia for a period of 1 day to 6 months, from one to five times daily.

### Prophetic Example 7 -Psoriasis

Psoriasis is a common skin condition which is characterized by an overactive immune response. By inhibiting harmful complement activation, low molecular weight ATA compounds will be effective therapeutics. Topically apply a composition comprising 0.1 to 100 mg/mL ATA, AQA, AHA or ATAC to affected areas of the skin a patient suffering from psoriasis for a period of 1 day to 6 months, from one to five times daily.

As will be apparent to those skilled in the art in the light of the foregoing disclosure, many alterations and modifications are possible in the practice of this invention without departing from the scope thereof. The scope of the claims should not be limited by the preferred embodiments set forth in the examples, but should be given the broadest interpretation consistent with the description as a whole.

## Claims

1. Aurin tricarboxylic acid (ATA), aurin quadracarboxylic acid (AQA), aurin hexacarboxylic acid (AHA), or a combination thereof for use as an active agent for topical treatment of an inflammatory skin condition, wherein said aurin tricarboxylic acid (ATA), aurin quadracarboxylic acid (AQA), aurin hexacarboxylic acid (AHA), and/or a combination thereof have a molecular weight less than 1kDa.

2. Aurin tricarboxylic acid (ATA), aurin quadracarboxylic acid (AQA), aurin hexacarboxylic acid (AHA), or a combination thereof for the use according to claim 1, wherein the active agent is ATA.

3. Aurin tricarboxylic acid (ATA), aurin quadracarboxylic acid (AQA), aurin hexacarboxylic acid (AHA), or a combination thereof for the use according to claim 1, wherein the active agent is AQA.

4. Aurin tricarboxylic acid (ATA), aurin quadracarboxylic acid (AQA), aurin hexacarboxylic acid (AHA), or a combination thereof for the use according to claim 1,wherein the active agent is AHA.

5. Aurin tricarboxylic acid (ATA), aurin quadracarboxylic acid (AQA), aurin hexacarboxylic acid (AHA), or a combination thereof for the use according to claim 1, wherein the active agent is the combination of ATA, AQA and AHA.

6. Aurin tricarboxylic acid (ATA), aurin quadracarboxylic acid (AQA), aurin hexacarboxylic acid (AHA), or a combination thereof for the use according to claim 5,wherein the combination consists of 60 to 90% ATA, 10 to 30% AQA, and 1 to 20% AHA.

7. Aurin tricarboxylic acid (ATA), aurin quadracarboxylic acid (AQA), aurin hexacarboxylic acid (AHA), or a combination thereof for the use according to claim 5, wherein the combination consists of 78% ATA, 15% AQA, and 7% AHA.

8. Aurin tricarboxylic acid (ATA), aurin quadracarboxylic acid (AQA), aurin hexacarboxylic acid (AHA), or a combination thereof for the use according to any one of claims 1 to 7, wherein said skin inflammatory condition is selected from the group consisting of: acne vulgaris, allergic or atopic dermatitis, alopecia areata, androgenetic alopecia, dandruff or seborrheic dermatitis, dermatitis herpetiformis, discoid lupus erythematosus, pemphigus, primary cicatricial alopecia, psoriasis, and a thermal or ultraviolet burn.

9. Aurin tricarboxylic acid (ATA), aurin quadracarboxylic acid (AQA), aurin hexacarboxylic acid (AHA), or a combination thereof for the use according to any one of claims 1 to 7, wherein the condition is any condition affecting the skin involving abnormal activation of complement.

10. Aurin tricarboxylic acid (ATA), aurin quadracarboxylic acid (AQA), aurin hexacarboxylic acid (AHA), or a combination thereof for the use according to any one of claims 1 to 9, wherein the active agent is formulated in a hair care preparation, a skin care preparation, or a pharmaceutical composition at a concentration of 0.1 to 100 mg/mL.

11. Aurin tricarboxylic acid (ATA), aurin quadracarboxylic acid (AQA), aurin hexacarboxylic acid (AHA), or a combination thereof for the use according to claim 10, wherein the hair care preparation, skin care preparation or pharmaceutical composition is formulated as spray, gel, cream, lotion, stick, ointment, scrub, soap bar, tonic, roll-on formulation, sunscreen, shampoo or mousse.

## Patentansprüche

1. Aurin-Tricarbonsäure (ATA), Aurin-Quadracarbonsäure (AQA), Aurin-Hexacarbonsäure (AHA) oder eine Kombination davon zur Verwendung als Wirkstoff zur topischen Behandlung einer entzündlichen Hauterkrankung, wobei die Aurin-Tricarbonsäure (ATA), Aurin-Quadracarbonsäure (AQA), Aurin-Hexacarbonsäure (AHA) und/oder eine Kombination davon haben ein Molekulargewicht von weniger als 1 kDa.

2. Aurin-Tricarbonsäure (ATA), Aurin-Quadracarbonsäure (AQA), Aurin-Hexacarbonsäure (AHA) oder eine Kombination davon zur Verwendung nach Anspruch 1, wobei der Wirkstoff ATA ist.

3. Aurin-Tricarbonsäure (ATA), Aurin-Quadracarbonsäure (AQA), Aurin-Hexacarbonsäure (AHA) oder eine Kombination davon zur Verwendung nach Anspruch 1, wobei der Wirkstoff AQA ist.

4. Aurin-Tricarbonsäure (ATA), Aurin-Quadracarbonsäure (AQA), Aurin-Hexacarbonsäure (AHA) oder eine Kombination davon zur Verwendung nach Anspruch 1, wobei der Wirkstoff AHA ist.

5. Aurin-Tricarbonsäure (ATA), Aurin-Quadracarbonsäure (AQA), Aurin-Hexacarbonsäure (AHA) oder eine Kombination davon zur Verwendung nach Anspruch 1, wobei der Wirkstoff die Kombination von ATA, AQA und AHA ist.

6. Aurin-Tricarbonsäure (ATA), Aurin-Quadracarbonsäure (AQA), Aurin-Hexacarbonsäure (AHA) oder eine Kombination davon zur Verwendung nach Anspruch 5, wobei die Kombination aus 60 bis 90% ATA, aus 10 bis 30% AQA und aus 1 bis 20% AHA besteht.

7. Aurin-Tricarbonsäure (ATA), Aurin-Quadracarbonsäure (AQA), Aurin-Hexacarbonsäure (AHA) oder eine Kombination davon zur Verwendung nach Anspruch 5, wobei die Kombination aus 78% ATA, aus 15% AQA und aus 7% AHA besteht.

8. Aurin-Tricarbonsäure (ATA), Aurin-Quadracarbonsäure (AQA), Aurin-Hexacarbonsäure (AHA) oder eine Kombination davon zur Verwendung nach einem der Ansprüche 1 bis 7, wobei die entzündliche Hauterkrankung aus der Gruppe ausgewählt wird, die aus Akne vulgaris, allergischer oder atopischer Dermatitis, Alopezie areata, androgenetischer Alopezie, Schuppen oder seborrhoischer Dermatitis, Dermatitis herpetiformis, diskoider Lupus erythematodes, Pemphigus, primärer narbigen Alopezie, Psoriasis und einer thermischen oder ultravioletten Verbrennung besteht.

9. Aurin-Tricarbonsäure (ATA), Aurin-Quadracarbonsäure (AQA), Aurin-Hexacarbonsäure (AHA) oder eine Kombination davon zur Verwendung nach einem der Ansprüche 1 bis 7, wobei die Erkrankung jede Erkrankung ist, die die Haut betrifft und eine anormale Aktivierung des Komplements umfasst.

10. Aurin-Tricarbonsäure (ATA), Aurin-Quadracarbonsäure (AQA), Aurin-Hexacarbonsäure (AHA) oder eine Kombination davon zur Verwendung nach einem der Ansprüche 1 bis 9, wobei der Wirkstoff in einer Haarpflegezubereitung, einer Hautpflegezubereitung oder einer pharmazeutischen Zusammensetzung in einer Konzentration von 0,1 bis 100 mg/ml formuliert wird.

11. Aurin-Tricarbonsäure (ATA), Aurin-Quadracarbonsäure (AQA), Aurin-Hexacarbonsäure (AHA) oder eine Kombination davon zur Verwendung nach Anspruch 10, wobei die Haarpflegezubereitung, die Hautpflegezubereitung oder die pharmazeutische Zusammensetzung als Spray, Gel, Creme, Lotion, Stick, Salbe, Peeling, Seife, Tonic, Roll-on Formulierung, Sonnencreme, Shampoo oder Mousse formuliert wird.

## Revendications

1. Acide aurine tricarboxylique (ATA), acide aurine quadracarboxylique (AQA), acide aurine hexacarboxylique (AHA) ou leur combinaison pour une utilisation en tant qu'agent actif pour le traitement topique d'une affection cutanée inflammatoire, où lesdits acide aurine tricarboxylique (ATA), acide aurine quadracarboxylique (AQA), acide aurine hexacarboxylique (AHA) et/ou leur combinaison ont un poids moléculaire inférieur à 1 kDa.

2. Acide aurine tricarboxylique (ATA), acide aurine quadracarboxylique (AQA), acide aurine hexacarboxylique (AHA) ou leur combinaison pour l'utilisation selon la revendication 1, où l'agent actif est ATA.

3. Acide aurine tricarboxylique (ATA), acide aurine quadracarboxylique (AQA), acide aurine hexacarboxylique (AHA) ou leur combinaison pour l'utilisation selon la revendication 1, où l'agent actif est AQA.

4. Acide aurine tricarboxylique (ATA), acide aurine quadracarboxylique (AQA), acide aurine hexacarboxylique (AHA) ou leur combinaison pour l'utilisation selon la revendication 1, où l'agent actif est AHA.

5. Acide aurine tricarboxylique (ATA), acide aurine quadracarboxylique (AQA), acide aurine hexacarboxylique (AHA) ou leur combinaison pour l'utilisation selon la revendication 1, où l'agent actif est la combinaison de ATA, AQA et AHA.

6. Acide aurine tricarboxylique (ATA), acide aurine quadracarboxylique (AQA), acide aurine hexacarboxylique (AHA) ou leur combinaison pour l'utilisation selon la revendication 5, où la combinaison est constituée de 60 à 90% d'ATA, de 10 à 30% d'AQA et de 1 à 20% d'AHA.

7. Acide aurine tricarboxylique (ATA), acide aurine quadracarboxylique (AQA), acide aurine hexacarboxylique (AHA) ou leur combinaison pour l'utilisation selon la revendication 5, où la combinaison est constituée de 78% d'ATA, de 15% d'AQA et de 7% d'AHA.

8. Acide aurine tricarboxylique (ATA), acide aurine quadracarboxylique (AQA), acide aurine hexacarboxylique (AHA) ou leur combinaison pour l'utilisation selon l'une quelconque des revendications 1 à 7, où ladite affection cutanée inflammatoire est choisie dans le groupe formé par: acné vulgaire, dermatite allergique ou atopique, alopécie areata, alopécie androgénétique, dermatite pelliculaire ou séborrhéique, dermatite herpétiforme, lupus érythémateux discoïde, pemphigus, alopécie cicatricielle primaire, psoriasis et une brûlure thermique ou ultraviolette.

9. Acide aurine tricarboxylique (ATA), acide aurine quadracarboxylique (AQA), acide aurine hexacarboxylique (AHA) ou leur combinaison pour l'utilisation selon l'une quelconque des revendications 1 à 7, où l'affection est n'importe quelle affection affectant la peau impliquant une activation anormale du complément.

10. Acide aurique tricarboxylique (ATA), acide aurique quadracarboxylique (AQA), acide aurine hexacarboxylique (AHA) ou leur combinaison pour l'utilisation selon l'une quelconque des revendications 1 à 9, où l'agent actif est formulé dans une préparation pour les soins des cheveux, une préparation pour les soins de la peau ou une composition pharmaceutique à une concentration de 0,1 à 100 mg/ml.

11. Acide aurine tricarboxylique (ATA), acide aurine quadracarboxylique (AQA), acide aurine hexacarboxylique (AHA) ou leur combinaison pour l'utilisation selon la revendication 10, où la préparation pour les soins des cheveux, la préparation pour les soins de la peau ou la composition pharmaceutique est formulée comme vaporisateur, gel, crème, lotion, stick, pommade, gommage, barre de savon, tonique, formule roll-on, crème solaire, shampoing ou mousse.
